(19)

Europäisches
Patentamt
European
Patent Office
Office européen
des brevets

(11)  **EP 1 814 581 B1**

(12)  **EUROPEAN PATENT SPECIFICATION**

(45) Date of publication and mention
of the grant of the patent:
**16.03.2016  Bulletin 2016/11**

(51) Int Cl.:
*A61K 38/28* (2006.01)        *A61K 38/26* (2006.01)
*A61K 47/26* (2006.01)        *A61K 47/10* (2006.01)
*A61P 3/10* (2006.01)

(21) Application number: **05803458.8**

(22) Date of filing: **11.11.2005**

(86) International application number:
**PCT/EP2005/055916**

(87) International publication number:
**WO 2006/051103 (18.05.2006 Gazette 2006/20)**

(54) **STABLE FORMULATIONS OF PEPTIDES COMPRISING AN ACYLATED GLP-1 ANALOGUE AND A BASAL INSULINE**

STABILE PEPTIDFORMULIERUNGEN ENTHALTEND EIN ACYLIERTES GLP-1 ANALOGON UND EIN BASALES INSULIN

PREPARATIONS STABLES DE PEPTIDES COMPRENANT UN ANALOGUE DU GLP-1 ACYLE ET UNE INSULINE BASALE

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.11.2004  DK 200401752
13.05.2005  EP 05104049
19.09.2005  DK 200500589**

(43) Date of publication of application:
**08.08.2007  Bulletin 2007/32**

(73) Proprietor: **NOVO NORDISK A/S
2880 Bagsværd (DK)**

(72) Inventors:
• **LUDVIGSEN, Svend
DK-3540 Lynge (DK)**
• **SCHLEIN, Morten
DK-2300 København S (DK)**
• **BØVING, Tine Elisabeth Gottschalk
DK-2800 Lyngby (DK)**

(56) References cited:
**WO-A-00/37098        WO-A-01/00223
WO-A-03/020201        WO-A-2005/046716**

EP 1 814 581 B1

## Description

## FIELD OF THE INVENTION

The present invention relates to the field of pharmaceutical formulations. More specifically the invention pertains to shelf-stable pharmaceutical formulations comprising insulinotropic peptide and insulin.

## BACKGROUND OF THE INVENTION

Diabetes mellitus is a metabolic disorder in which the ability to utilize glucose is partly or completely lost. About 5% of all people suffer from diabetes and the disorder approaches epidemic proportions. Since the introduction of insulin in the 1920's, continuous efforts have been made to improve the treatment of diabetes mellitus. Since people suffering from diabetes are subject to chronic treatment over several decades, there is a major need for safe, convenient and life quality improving insulin formulations.

In the treatment of diabetes mellitus, many varieties of insulin formulations have been suggested and used, such as regular insulin, isophane insulin (designated NPH), insulin zinc suspensions (such as Semilente®, Lente®, and Ultralente®), and biphasic isophane insulin. Some of the commercial available insulin formulations are characterized by a fast onset of action and other formulations have a relatively slow onset but show a more or less prolonged action. Fast-acting insulin formulations are usually solutions of insulin, while retarded acting insulin formulations can be suspensions containing insulin in crystalline and/or amorphous form precipitated by addition of zinc salts alone or by addition of protamine or by a combination of both.

Normally, insulin formulations are administered by subcutaneous injection. What is important for the patient is the action profile of the insulin formulation which is the action of insulin on the glucose metabolism as a function of the time from the injection. In this profile, inter alia, the time for the onset, the maximum value, and the total duration of action are important. A variety of insulin formulations with different action profiles are desired and requested by the patients.

Human insulin consists of two polypeptide chains, the so-called A and B chains which contain 21 and 30 amino acid residues, respectively. The A and B chains are interconnected by two cysteine disulphide bridges. Insulin from most other species has a similar construction, but may not contain the same amino acid residues at the same positions. Within the last decade a number of human insulin analogues have been developed. They are designed for particular profiles of action, i.e. fast acting or prolonged action.

Another peptide expected to become very important in the treatment of diabetes is glucagon-like peptide-1 (GLP-1). Human GLP-1 is a 37 amino acid residue peptide originating from preproglucagon which is synthesized i.a. in the L-cells in the distal ileum, in the pancreas and in the brain. GLP-1 is an important gut hormone with regulatory function in glucose metabolism and gastrointestinal secretion and metabolism. GLP-1 stimulates insulin secretion in a glucose-dependant manner, stimulates insulin biosynthesis, promotes beta cell rescue, decreases glucagon secretion, gastric emptying and food intake. A simple system is used to describe fragments and analogues of this peptide. Thus, for example, Gly$^8$-GLP-1 (7-37) designates an analogue of GLP-1 (7-37) formally derived from GLP-1 (7-37) by substituting the naturally occurring amino acid residue in position 8 (Ala) by Gly. Similarly, Lys$^{34}$(N$^\varepsilon$-tetradecanoyl)-GLP-1(7-37) designates GLP-1 (7-37) wherein the ε-amino group of the Lys residue in position 34 has been tetradecanoylated. PCT publications WO 98/08871 and WO 99/43706 disclose stable derivatives of GLP-1 analogues, which have a lipophilic substituent. These stable derivatives of GLP-1 analogues have a protracted profile of action compared to the corresponding GLP-1 analogues. Apart from GLP-1 peptides, the lizard peptide exendin-4 also has a strong insulinotropic effect.

As the type 2 diabetes population is rapidly increasing in the world, there is a much larger need for simpler administration of more effective drugs. The effects of GLP-1 are expected to give patients a very effective and safe lowering of blood glucose. However, some patients may benefit from an extra basal insulin to help normalise post prandial blood glucose levels. A combination formulation comprising a basal insulin and an insulinotropic peptide, may be a very efficacious treatment as well as one requiring less injections of the patient. Because only a low dose of insulin is given with the injection and the GLP-1 counterpart of the formulation controls glucose for the rest of the day and night, and since GLP-1 does not lead to hypoglycaemia it may also be a very safe treatment.

Thus, there is a big need for shelf-stable pharmaceutical compositions comprising an insulinotropic peptide and a basal insulin in one combined formulation.

## BREIF DESCRIPTION OF THE DRAWINGS

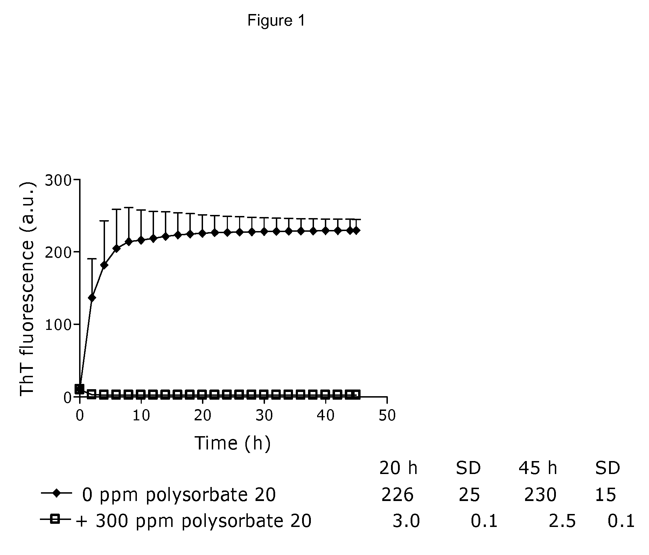

Figure 1. Mixtures of detemir 2.4 mM and liraglutide 1.2 mM in a formulation comprising 1.2 mM Zn acetate, 5 mM glycyl glycine, 20 mM sodium chloride, 60 mM phenol and 14 mg/ml propylene glycol at pH 7.7 (diamond) and the same formulation but with the addition of 300 ppm polysorbate 20 (tween 20).

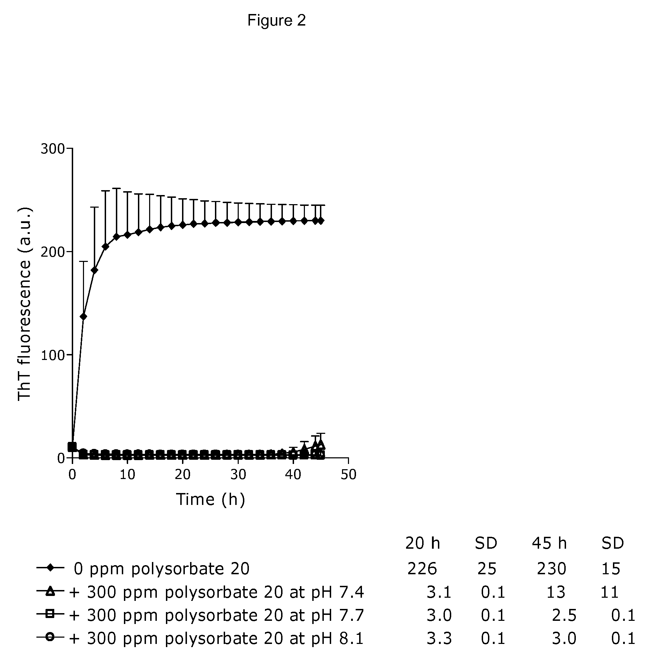

Figure 2. Mixtures of detemir 2.4 mM and liraglutide 1.2 mM in a formulation comprising 1.2 mM Zn acetate, 5 mM glycyl glycine, 20 mM sodium chloride, 60 mM phenol and 14 mg/ml propylene glycol at pH 7.7 (diamond) and the same formulation but with the addition of 300 ppm polysorbate 20 (tween 20) at three different pH values, triangles at pH 7.4, squares at pH 7.7 and circles at pH 8.1.

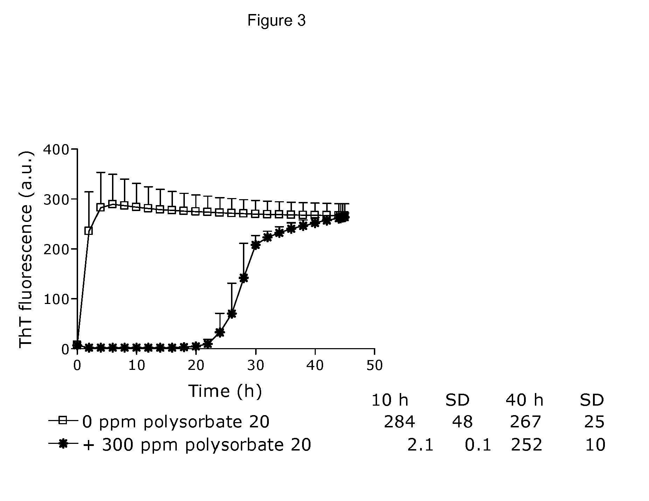

Figure 3. Mixtures of detemir 2.4 mM and liraglutide 2.4 mM in a formulation comprising 1.2 mM Zn acetate, 8 mM sodium phosphate buffer, 20 mM sodium chloride, 60 mM phenol and 14 mg/ml propylene glycol at pH 7.7 (squares) and the same formulation but with the addition of 300 ppm polysorbate 20 shown with star symbols.

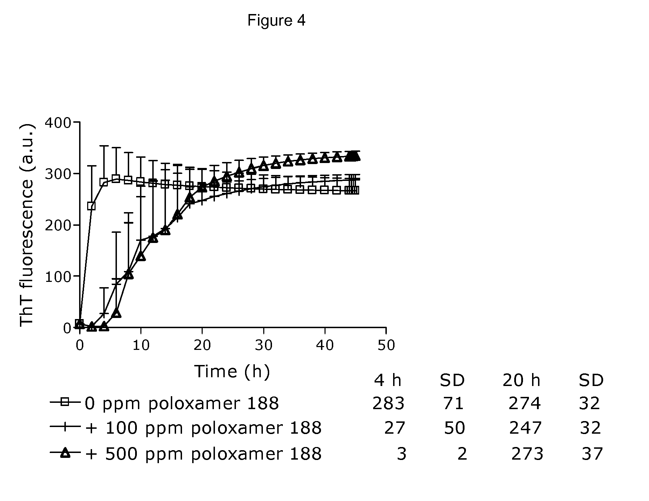

Figure 4. Mixtures of detemir 2.4 mM and liraglutide 2.4 mM in a formulation comprising 1.2 mM Zn acetate, 8 mM sodium phosphate buffer, 20 mM sodium chloride, 60 mM phenol and 14 mg/ml propylene glycol at pH 7.7 (square) and the same formulation but with the addition of 2 levels of poloxamer 188, 100 ppm shown with crosses and 500 ppm with triangles.

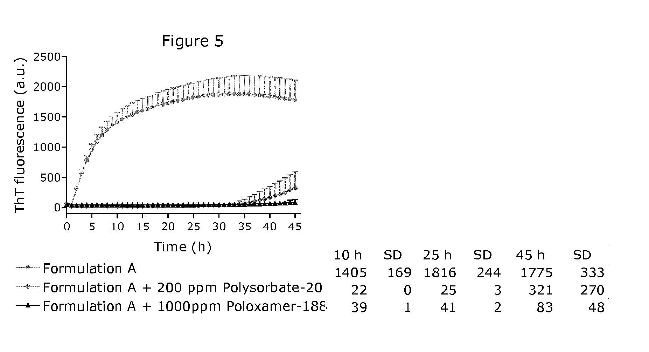

Figure 5. Formulation A consists of: 0.6 mM insulin $N^{\varepsilon B29}$-($N^{\alpha}$(HOOC(CH$_2$)$_{14}$CO)-y-Glu) desB30 human insulin, 0.3 mM Zn$^{2+}$ (corresponding to 3 Zn$^{2+}$ ions per insulin hexamer), 60 mM phenol, 14 mg/ml propylene glycol, 5 mM phosphate pH 7.7 and 1.2 mM liraglutide and the same formulation with the addition of either 200 ppm Polysorbate-20 or 1000 ppm Poloxamer-188.

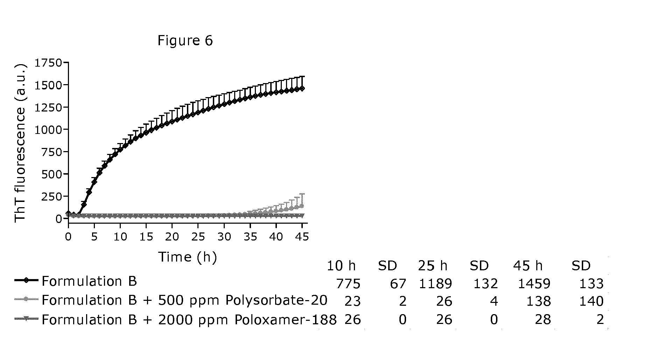

Figure 6. Formulation B consists of: 0.6 mM $N^{\varepsilon B29}$-($N\alpha$-(HOOC(CH$_2$)$_{14}$CO)-$\gamma$-Glu) desB30 human insulin, 0.5 mM Zn$^{2+}$ (corresponding to 5 Zn$^{2+}$ ions per insulin hexamer), 60 mM phenol, 14 mg/ml propylene glycol, 5 mM phosphate pH 7.7 and 1.2 mM liraglutide. Also shown in the addition of 2000 ppm Poloxamer-188.

**DESCRIPTION OF THE INVENTION**

The following is a detailed definition of the terms used in the specification.

The term "shelf-stable pharmaceutical composition" as used herein means a pharmaceutical composition which is stable for at least the period which is required by regulatory agencies in connection with therapeutic proteins. Preferably, a shelf-stable pharmaceutical composition is stable for at least one year at 5 °C. Stability includes chemical stability as well as physical stability.

The term "effective amount" as used herein means a dosage which is sufficient in order for the treatment of the patient to be effective compared with no treatment.

The term "medicament" as used herein means a pharmaceutical composition suitable for administration of the pharmaceutically active compounds to a patient.

The term "pharmaceutical composition" as used herein means a product comprising an active compound or a salt thereof together with pharmaceutical excipients such as buffer, preservative and tonicity modifier, said pharmaceutical composition being useful for treating, preventing or reducing the severity of a disease or disorder by administration of said pharmaceutical composition to a person. Thus a pharmaceutical composition is also known in the art as a pharmaceutical formulation. It is to be understood that pH of a pharmaceutical composition which is to be reconstituted is the pH value which is measured on the reconstituted composition produced by reconstitution in the prescribed reconstitution liquid at room temperature.

The term "pharmaceutically acceptable" as used herein means suited for normal pharmaceutical applications, i.e. giving rise to no adverse events in patients etc.

The term "buffer" as used herein refers to a chemical compound in a pharmaceutical composition that reduces the tendency of pH of the composition to change over time as would otherwise occur due to chemical reactions. Buffers include chemicals such as sodium phosphate, TRIS, glycine and sodium citrate.

The term "preservative" as used herein refers to a chemical compound which is added to a pharmaceutical composition to prevent or delay microbial activity (growth and metabolism). Examples of pharmaceutically acceptable preservatives are phenol, m-cresol and a mixture of phenol and m-cresol.

The term "isotonicity agent" as used refers to a chemical compound in a pharmaceutical composition that serves to modify the osmotic pressure of the pharmaceutical composition so that the osmotic pressure becomes closer to that of human plasma. Isotonicity agents include NaCl, glycerol, mannitol etc.

The term "stabilizer" as used herein refers to chemicals added to peptide containing pharmaceutical compositions in order to stabilize the peptide, i.e. to increase the shelf life and/or in-use time of such compositions. Examples of stabilizers used in pharmaceutical formulations are L-glycine, L-histidine, arginine, polyethylene glycol, and carboxymethylcellulose.

The term "Surfactant" as used herein refers to any molecules or ions that are comprised of a water-soluble (hydrophilic) part, the head, and a fat-soluble (lipophilic) segment. Surfactants accumulate preferably at interfaces, which the hydrophilic part is orientated towards the water (hydrophilic phase) and the lipophilic part towards the oil- or hydrophobic phase (*i.e.* glass, air, oil etc.). The concentration at which surfactants begin to form micelles is known as the critical micelle concentration or CMC. Furthermore, surfactants lower the surface tension of a liquid. Surfactants are also known as amphipathic compounds. The term "Detergent" is a synonym used for surfactants in general.

**Anionic** surfactants may be selected from the group of: Chenodeoxycholic acid, Chenodeoxycholic acid sodium salt, Cholic acid, Dehydrocholic acid, Deoxycholic acid, Deoxycholic acid methyl ester, Digitonin, Digitoxigenin, N,N-Dimethyldodecylamine N-oxide, Docusate sodium, Glycochenodeoxycholic acid sodium, Glycocholic acid hydrate, Glycodeoxycholic acid monohydrate, Glycodeoxycholic acid sodium salt, Glycodeoxycholic acid sodium salt, Glycolithocholic acid 3-sulfate disodium salt, Glycolithocholic acid ethyl ester, N-Lauroylsarcosine sodium salt, N-Lauroylsarcosine sodium salt, N-Lauroylsarcosine, N-Lauroylsarcosine, Lithium dodecyl sulfate, Lugol, 1-Octanesulfonic acid sodium salt, 1-Octanesulfonic acid sodium salt, Sodium 1-butanesulfonate, Sodium 1-decanesulfonate, Sodium 1-dodecanesulfonate, Sodium 1-heptanesulfonate, Sodium 1-heptanesulfonate, Sodium 1-nonanesulfonate, Sodium 1-propanesulfonate monohydrate, Sodium 2-bromoethanesulfonate, Sodium cholate hydrate, ox or sheep bile, Sodium cholate hydrate, Sodium choleate, Sodium deoxycholate, Sodium dodecyl sulfate, Sodium dodecyl sulfate, Sodium hexanesulfonate, Sodium octyl sulfate, Sodium pentanesulfonate, Sodium taurocholate, Taurochenodeoxycholic acid sodium salt, Taurodeoxycholic acid sodium salt monohydrate, Taurolithocholic acid 3-sulfate disodium salt, Tauroursodeoxycholic acid sodium salt, Trizma® dodecyl sulfate, DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), Dodecylphosphocholine (FOS-Choline-12), Decylphosphocholine (FOS-Choline-10), Nonylphosphocholine (FOS-Choline-9), dipalmitoyl phosphatidic acid, sodium caprylate, and/or Ursodeoxycholic acid.

**Cationic** surfactants may be selected from the group of: Alkyltrimethylammonium bromide

Benzalkonium chloride, Benzalkonium chloride, Benzyldimethylhexadecylammonium chloride, Benzyldimethyltetradecylammonium chloride, Benzyltrimethylammonium tetrachloroiodate, Dimethyldioctadecylammonium bromide, Dodecylethyldimethylammonium bromide, Dodecyltrimethylammonium bromide, Dodecyltrimethylammonium bromide, Ethylhexadecyldimethylammonium bromide, Hexadecyltrimethylammonium bromide, Hexadecyltrimethylammonium bromide, Polyoxyethylene(10)-N-tallow-1,3-diaminopropane, Thonzonium bromide, and/or Trimethyl(tetradecyl)ammonium bromide.

**Nonionic** surfactants may be selected from the group of: BigCHAP, Bis(polyethylene glycol bis[imidazoyl carbonyl]), block copolymers as polyethyleneoxide/polypropyleneoxide block copolymers such as poloxamers, poloxamer 188 and poloxamer 407, Brij® 35, Brij® 56, Brij® 72, Brij® 76, Brij® 92V, Brij® 97, Brij® 58P, Cremophor® EL, Decaethylene glycol monododecyl ether, N-Decanoyl-N-methylglucamine, n-Dodecanoyl-N-methylglucamide, alkyl-polyglucosides, ethoxylated castor oil, Heptaethylene glycol monodecyl ether, Heptaethylene glycol monododecyl ether, Heptaethylene glycol monotetradecyl ether, Hexaethylene glycol monododecyl ether, Hexaethylene glycol monohexadecyl ether, Hexaethylene glycol monooctadecyl ether, Hexaethylene glycol monotetradecyl ether, Igepal CA-630, Igepal CA-630, Methyl-6-O-(N-heptylcarbamoyl)-beta-D-glucopyranoside, Nonaethylene glycol monododecyl ether, N-Nonanoyl-N-methylglucamine, N-Nonanoyl-N-methylglucamine, Octaethylene glycol monodecyl ether, Octaethylene glycol monododecyl ether, Octaethylene glycol monohexadecyl ether, Octaethylene glycol monooctadecyl ether, Octaethylene glycol monotetradecyl ether, Octyl-β-D-glucopyranoside, Pentaethylene glycol monodecyl ether, Pentaethylene glycol monododecyl ether, Pentaethylene glycol monohexadecyl ether, Pentaethylene glycol monohexyl ether, Pentaethylene glycol monooctadecyl ether, Pentaethylene glycol monooctyl ether, Polyethylene glycol diglycidyl ether, Polyethylene glycol ether W-1, Polyoxyethylene 10 tridecyl ether, Polyoxyethylene 100 stearate, Polyoxyethylene 20 isohexadecyl ether, Polyoxyethylene 20 oleyl ether, Polyoxyethylene 40 stearate, Polyoxyethylene 50 stearate, Polyoxyethylene 8 stearate, Polyoxyethylene bis(imidazolyl carbonyl), Polyoxyethylene 25 propylene glycol stearate, Saponin from Quillaja bark, Span® 20, Span® 40, Span® 60, Span® 65, Span® 80, Span® 85, Tergitol, Type 15-S-12, Tergitol, Type 15-S-30, Tergitol, Type 15-S-5, Tergitol, Type 15-S-7, Tergitol, Type 15-S-9, Tergitol, Type NP-10, Tergitol, Type NP-4, Tergitol, Type NP-40, Tergitol, Type NP-7, Tergitol, Type NP-9, Tetradecyl-β-Dmaltoside, Tetraethylene glycol monodecyl ether, Tetraethylene glycol monododecyl ether, Tetraethylene glycol monotetradecyl ether, Triethylene glycol monodecyl ether, Triethylene glycol monododecyl ether, Triethylene glycol monohexadecyl ether, Triethylene glycol monooctyl ether, Triethylene glycol monotetradecyl ether, Triton CF-21, Triton CF-32, Triton DF-12, Triton DF-16, Triton GR-5M, Triton QS-15, Triton QS-44, Triton X-100, Triton X-102, Triton X-15, Triton X-151, Triton X-200, Triton X-207, Triton® X-100, Triton® X-114, Triton® X-165 solution, Triton® X-305 solution, Triton® X-405, Triton® X-45, Triton® X-705-70, TWEEN® 20, TWEEN® 40, TWEEN® 60, TWEEN® 6, TWEEN® 65, TWEEN® 80, TWEEN® 81, TWEEN® 85, Tyloxapol, sphingophospholipids (sphingomyelin), and sphingoglycolipids (ceramides, gangliosides), phospholipids, and/or n-Undecyl β-D-glucopyranoside.

**Zwitterionic** surfactants may be selected from the group of: CHAPS, CHAP-SO, 3-(Decyldimethylammonio)propanesulfonate inner salt, 3-(Dodecyldimethylammonio)propanesulfonate inner salt, 3-(Dodecyldimethylammonio)propanesulfonate inner salt, 3-(N,N-Dimethylmyristylammonio)propanesulfonate, 3-(N,N-Dimethyloctadecylammonio)-propanesulfonate, 3-(N,N-Dimethyloctylammonio)propanesulfonate inner salt, 3-(N,N-Dimethylpalmitylammonio)propanesulfonate, N-alkyl-N,N-dimethylammonio-1-propanesulfonates, 3-cholamido-1-propyldimethylammonio-1-propanesulfonate, Dodecylphosphocholine, myristoyl lysophosphatidylcholine, Zwittergent 3-12 (N-dodecyl-N,N-dimethyl-3-ammonio-1-propanesulfonate), Zwittergent 3-10 (3-(Decyldimethyl-ammonio)propanesulfonate inner salt), Zwittergent 3-08 (3-(Octyldimethyl-ammonio)propanesulfonate), glycerophospholipids (lecithins, kephalins, phosphatidyl serine), glyceroglycolipids (galactopyranoside), alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, lysophosphatidylserine and lysophosphati-dylthreonine, acylcarnitines and derivatives, $N^{beta}$-acylated derivatives of lysine, arginine or histidine, or side-chain acylated derivatives of lysine or arginine, $N^{beta}$-acylated derivatives of dipeptides comprising any combination of lysine, arginine or histidine and a neutral or acidic amino acid, $N^{beta}$-acylated derivative of a tripeptide comprising any combination of a neutral amino acid and two charged amino acids, or the surfactant may be selected from the group of imidazoline derivatives, long-chain fatty acids and salts thereof $C_6$-$C_{12}$ (eg. oleic acid and caprylic acid), N-Hexadecyl-N,N-dimethyl-3-ammonio-1-propanesulfonate, anionic (alkyl-aryl-sulphonates) monovalent surfactants, palmitoyl lysophosphatidyl-L-serine, lysophospholipids (e.g. 1-acyl-sn-glycero-3-phosphate esters of ethanolamine, choline, serine or threonine), or mixtures thereof.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "insulin peptide" as used herein means a peptide which is either human insulin or a chemically modified human insulin, such as an analog or a derivative thereof.

The term "human insulin" as used herein means the human hormone whose structure and properties are well known. Human insulin has two polypeptide chains that are connected by disulphide bridges between cysteine residues, namely the A-chain and the B-chain. The A-chain is a 21 amino acid peptide and the B-chain is a 30 amino acid peptide, the two chains being connected by three disulphide bridges : one between the cysteines in position 6 and 11 of the A-chain, the second between the cysteine in position 7 of the A-chain and the cysteine in position 7 of the B-chain, and the third between the cysteine in position 20 of the A-chain and the cysteine in position 19 of the B-chain.

The term "meal-related insulin peptide" as used herein means an insulin peptide which has a time-action of less than 8 hours in standard models of diabetes. Preferably, the meal-related human insulin has a time-action of less than about 5 hours. Preferably, the meal-related insulin has a time-action in the range from 0 hours to about 4 hours. Preferably, the meal-related insulin has a time-action similar to that observed for commercial pharmaceutical compositions of Actrapid®, Novolog®, and Humalog®. The term about in relation to the time-action of insulins means + or - 30 minutes.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been deleted from the peptide and or wherein one or more amino acid residues have been added to the peptide. Such addition or deletion of amino acid residues can take place at the N-terminal of the peptide and/or at the C-terminal of the peptide. In one embodiment an analogue comprises less than 6 modifications (substitutions, deletions, additions) relative to the native peptide. In another embodiment an analogue comprises less than 5 modifications (substitutions, deletions, additions) relative to the native peptide. In another embodiment an analogue comprises less than 4 modifications (substitutions, deletions, additions) relative to the native peptide. In another embodiment an analogue comprises less than 3 modifications (substitutions, deletions, additions) relative to the native peptide. In another embodiment an analogue comprises less than 2 modifications (substitutions, deletions, additions) relative to the native peptide. In another embodiment an analogue comprises only a single modification (substitutions, deletions, additions) relative to the native peptide.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like. Examples of derivatives of human insulin are threonine methyl ester$^{B30}$ human insulin and $N^{\epsilon B29}$-tetradecanoyl des(B30) human insulin.

The term "basal insulin" as used herein means an insulin peptide which has a time-action of more than 8 hours in standard models of diabetes. Preferably, the basal insulin has a time-action of at least 9 hours. Preferably, the basal insulin has a time-action of at least 10 hours. Preferably, the basal meal-related insulin has a time-action in the range from 9 to 15 hours. Preferably, the meal-related insulin has a time-action similar to that observed for commercial pharmaceutical compositions of NPH insulin and $N^{\epsilon B29}$-tetradecanoyl des(B30) human insulin.

The term "GLP-1 compound" as used herein means GLP-1(7-37) (SEQ ID NO. 1), insulinotropic analogue thereof and insulinotropic derivatives thereof. Non-limiting examples of GLP-1 analogues are GLP-1 (7-36) amide, Arg[34]-GLP-1(7-37), Gly[8]-GLP-1 (7-37), Val[8]-GLP-1 (7-36)-amide and Val[8]Asp[22]-GLP-1(7-37). Non-limiting examples of GLP-1 derivatives are desamino-His[7], Arg[26], Lys[34]($N^\varepsilon$-($\gamma$-Glu($N^\alpha$-hexadecanoyl)))-GLP-1(7-37), desamino-His[7], Arg[26], Lys[34]($N^\varepsilon$-octanoyl)-GLP-1(7-37), Arg[26,34], Lys[38]($N^\varepsilon$-($\omega$-carboxypentadecanoyl))-GLP-1(7-38), Arg[26,34], Lys[36]($N^\varepsilon$-($\gamma$-Glu($N^\alpha$-hexadecanoyl)))-GLP-1(7-36) and Arg[34], Lys[26]($N^\varepsilon$-($\gamma$-Glu($N^\alpha$-hexadecanoyl)))-GLP-1(7-37).

The term "dipeptidyl aminopeptidase IV protected" as used herein in relation to an insulinotropic peptide means an insulinotropic peptide which is more resistant to the plasma peptidase dipeptidyl aminopeptidase IV (DPP-IV) than the native insulinotropic peptide. Resistance of an insulinotropic peptide towards degradation by dipeptidyl aminopeptidase IV is determined by the following degradation assay :

Aliquots of the insulinotropic peptide (5 nmol) are incubated at 37°C with 1 $\mu$L of purified dipeptidyl aminopeptidase IV corresponding to an enzymatic activity of 5 mU for 10-180 minutes in 100 $\mu$L of 0.1 M triethylamine-HCl buffer, pH 7.4. Enzymatic reactions are terminated by the addition of 5 $\mu$L of 10% trifluoroacetic acid, and the peptide degradation products are separated and quantified using HPLC analysis. One method for performing this analysis is : The mixtures are applied onto a Vydac C18 widepore (30 nm pores, 5 $\mu$m particles) 250 x 4.6 mm column and eluted at a flow rate of 1 ml/min with linear stepwise gradients of acetonitrile in 0.1 % trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Peptides and their degradation products may be monitored by their absorbance at 220 nm (peptide bonds) or 280 nm (aromatic amino acids), and are quantified by integration of their peak areas related to those of standards. The rate of hydrolysis of an insulinotropic peptide by dipeptidyl aminopeptidase IV is estimated at incubation times which result in less than 10% of the GLP-1 compound being hydrolysed.

The term "insulinotropic" as used herein referring to a peptide means the ability to stimulate secretion of insulin in response to an increased plasma glucose level. Insulinotropic peptides and compounds are agonists of the GLP-1 receptor. The insulinotropic property of a compound may be determined by in vitro or in vivo assays known in the art. The following in vitro assay may be used to determine the insulinotropic nature of a compound such as a peptide. Preferably insulinotropic compounds exhibit an $EC_{50}$ value in below assay of less than 5 nM, even more preferably EC50 values less than 500 pM.

Baby hamster kidney (BHK) cells expressing the cloned human GLP-1 receptor (BHK 467-12A) are grown in DMEM media with the addition of 100 IU/mL penicillin, 100 $\mu$L/mL streptomycin, 10% foetal calf serum and 1 mg/mL Geneticin G-418 (Life Technologies). Plasma membranes are prepared by homogenization in buffer (10 mM Tris-HCl, 30 mM NaCl and 1 mM dithiothreitol, pH 7.4, containing, in addition, 5 mg/mL leupeptin (Sigma), 5 mg/L pepstatin (Sigma), 100 mg/L bacitracin (Sigma), and 16 mg/L aprotinin (Calbiochem-Novabiochem, La Jolla, CA)). The homogenate was centrifuged on top of a layer of 41% W7v sucrose. The white band between the two layers was diluted in buffer and centrifuged. Plasma membranes were stored at -80 °C until used.

The functional receptor assay is carried out by measuring cAMP as a response to stimulation by the insulinotropic peptide or insulinotropic compound. Incubations are carried out in 96-well microtiter plates in a total volume of 140 mL and with the following final concentrations: 50 mM Tris-HCl, 1 mM EGTA, 1.5 mM $MgSO_4$, 1.7 mM ATP, 20 mM GTP, 2 mM 3-isobutyl-1-methylxanthine (IBMX), 0.01% w/v Tween-20, pH 7.4. Compounds are dissolved and diluted in buffer. GTP is freshly prepared for each experiment: 2.5 $\mu$g of membrane is added to each well and the mixture is incubated for 90 min at room temperature in the dark with shaking. The reaction is stopped by the addition of 25 mL 0.5 M HCl. Formed cAMP is measured by a scintillation proximity assay (RPA 542, Amersham, UK). A dose-response curves is plotted for the compound and the $EC_{50}$ value is calculated using GraphPad Prism software.

In one aspect the invention provides a shelf-stable pharmaceutical composition comprising an acylated GLP-1 analogue and a basal insulin, a pharmaceutically acceptable preservative, non-ionic surfactant, at a concentration of from 10 mg/L to 500 mg/L, and optionally a pharmaceutically acceptable tonicity modifier, where said composition has a pH that is in the range from 7.0 to 8.5, wherein said acylated GLP-1 analogue is Arg[34], Lys[26]($N^\varepsilon$-($\gamma$-Glu($N^\alpha$-hexadecanoyl)))-GLP-1(7-37) and said basal insulin is $N^{\varepsilon B29}$-tetradecanoyl des(B30) human insulin or $N^{\varepsilon B29}$-($N^\alpha$-(HOOC($CH_2$)$_{14}$CO)-$\gamma$Glu) desB30 human insulin. An embodiment of the invention comprises a shelf-stable pharmaceutical composition comprising an acylated GLP-1 analogue, a basal insulin, a pharmaceutically acceptable preservative, non-ionic surfactant at a concentration of from 10 mg/L to 500 mg/L, and optionally a pharmaceutically acceptable tonicity modifier, where said composition has a pH that is in the range from 7.0 to 8.5, wherein said acylated GLP-1 analogue is Arg[34], Lys[26]($N^\varepsilon$-($\gamma$-Glu($N^\alpha$-hexadecanoyl)))-GLP-1(7-37) and said basal insulin is $N^{\varepsilon B29}$-tetradecanoyl des(B30) human insulin or $N^{\varepsilon B29}$-($N^\alpha$(HOOC($CH_2$)$_{14}$CO)-$\gamma$-Glu) desB30 human insulin.

An embodiment of the invention comprises a pharmaceutical composition according to the above embodiments wherein the concentration of surfactant is from 20 mg/L to 400 mg/L.

An embodiment of the invention comprises a pharmaceutical composition according to any of the above embodiments, wherein the concentration of surfactant is from 20 mg/L to 300 mg/L.

An embodiment of the invention comprises a pharmaceutical composition according to any one of the above embodiments, wherein the concentration of surfactant is from 50 mg/L to 200 mg/L.

An embodiment of the invention comprises a pharmaceutical composition according to any one of the above embodiments, wherein the surfactant is poloxamer 188.

An embodiment of the invention comprises a pharmaceutical composition according to any one of the above embodiments, wherein the surfactant is selected from the group consisting of poloxamer 407, poloxamer 124, poloxamer 181, poloxamer 182, poloxamer 237, poloxamer 331 and poloxamer 338.

An embodiment of the invention comprises a pharmaceutical composition according to any one of the above embodiments, wherein the surfactant is polysorbate 20.

An embodiment of the invention comprises a composition according to the above embodiment, wherein said composition has a pH that is in the range from 7.0 to 8.5

An embodiment of the invention comprises a pharmaceutical composition according to any one of the above embodiments, further comprising an additional surfactant.

An embodiment of the invention comprises a pharmaceutical composition according to the above embodiment, wherein at least one surfactant is a non-ionic surfactant.

An embodiment of the invention comprises a pharmaceutical composition according to the above embodiments, wherein two different surfactants are non-ionic surfactants.

An embodiment of the invention comprises a pharmaceutical composition according to any one of the above embodiments, wherein all surfactants are non-ionic surfactants.

An embodiment of the invention comprises a pharmaceutical composition according to any one of the above embodiments, comprising poloxamer 188 and polysorbate 20 or tween 80.

An embodiment of the invention comprises a pharmaceutical composition according to any one of the previous embodiments, wherein pH is in the range from 7.4 to 8.0.

An embodiment of the invention comprises a pharmaceutical composition according to any one of the previous embodiments, comprising a buffer which is a phosphate buffer.

An embodiment of the invention comprises a pharmaceutical composition according to any one of the above embodiments, comprising a buffer which is a zwitterionic buffer.

An embodiment of the invention comprises a pharmaceutical composition according to the above embodiment, wherein the buffer is selected from the group consisting of glycyl-glycine, TRIS, bicine, HEPES, MOBS, MOPS, TES and mixtures thereof.

An embodiment of the invention comprises a pharmaceutical composition according to any one of the previous embodiments, wherein the tonicity modifier is selected from the group consisting of glycerol, propylene glycol and mannitol.

An embodiment of the invention comprises a pharmaceutical composition according to any one of the previous embodiments, wherein the preservative is selected from the group consisting of phenol, m-cresol, methyl p-hydroxybenzoate, propyl p-hydroxybenzoate, 2-phenoxyethanol, butyl p-hydroxybenzoate, 2-phenylethanol, benzyl alcohol, chlorobutanol, thiomerosal and mixtures thereof.

An embodiment of the invention comprises a pharmaceutical composition according to any one of the previous embodiments, wherein the concentration of said acylated GLP-1 analogue is in the range from 0.1 mg/ml to 25 mg/ml, in the range from 1 mg/ml to 25 mg/ml, in the range from 2 mg/ml to 15 mg/ml, in the range from 3 mg/ml to 10 mg/ml, or in the range from 3 mg/ml to 5 mg/ml.

An embodiment of the invention comprises a pharmaceutical composition according to any one of the above embodiments, wherein the concentration of said acylated GLP-1 analogue in the pharmaceutical composition is from $5\mu g/mL$ to 10mg/mL, from $5\mu g/mL$ to 5mg/mL, from $5\mu g/mL$ to 5mg/mL, from 0.1 mg/mL to 3mg/mL, or from 0.2mg/mL to 1 mg/mL.

An embodiment of the invention comprises a pharmaceutical composition according to the above embodiment, wherein said basal insulin is $N^{\varepsilon B29}$-tetradecanoyl des(B30) human insulin or $N^{\varepsilon B29}$-($N^{\alpha}$-(HOOC(CH$_2$)$_{14}$CO)-$\gamma$-Glu) desB30 human insulin.

An embodiment of the invention comprises a pharmaceutical composition according to any one of the previous embodiments, wherein the concentration of said basal insulin is in the range from 1.6 mg/mL to 5.6 mg/mL, or from 2.6 mg/mL to 4.6 mg/mL, or from 3.2 mg/mL to 4.0 mg/mL.

An embodiment of the invention comprises a method for preparation of a pharmaceutical composition according to any one of the previous embodiments, comprising dissolving said insulinotropic peptide and admixing the preservative and tonicity modifier, and finally admixing the dissolved basal insulin.

An embodiment of the invention comprises a method for preparation of a pharmaceutical composition according to any one of the above embodiments, comprising dissolving or suspending said basal insulin and admixing the preservative and tonicity modifier, and finally admixing the dissolved insulinotropic peptide.

An embodiment of the invention comprises the use of the pharmaceutical composition according to any one of the above embodiments for the preparation of a medicament for the treatment of hyperglycemia comprising parenteral administration to a mammal in need of such treatment.

An embodiment of the invention comprises the use of the pharmaceutical composition according to any one of the above embodiments for the preparation of a medicament for the treatment of obesity, beta-cell deficiency, IGT or dyslipidemia comprising parenteral administration to a mammal in need of such treatment.

The use of excipients such as preservatives, isotonic agents and surfactants in pharmaceutical compositions is well-known to the skilled person. For convenience reference is made to Remington: The Science and Practice of Pharmacy, 19th edition, 1995.

The parent glucagon-like peptide can be produced by peptide synthesis, e.g. solid phase peptide synthesis using t-Boc or F-Moc chemistry or other well established techniques. The parent glucagon-like peptide can also be produced by a method which comprises culturing a host cell containing a DNA sequence encoding the polypeptide and capable of expressing the polypeptide in a suitable nutrient medium under conditions permitting the expression of the peptide, after which the resulting peptide is recovered from the culture.

The medium used to culture the cells may be any conventional medium suitable for growing the host cells, such as minimal or complex media containing appropriate supplements. Suitable media are available from commercial suppliers or may be prepared according to published recipes (*e.g.* in catalogues of the American Type Culture Collection). The peptide produced by the cells may then be recovered from the culture medium by conventional procedures including separating the host cells from the medium by centrifugation or filtration, precipitating the proteinaceous components of the supernatant or filtrate by means of a salt, *e.g.* ammonium sulphate, purification by a variety of chromatographic procedures, *e.g.* ion exchange chromatography, gel filtration chromatography, affinity chromatography, or the like, dependent on the type of peptide in question.

The DNA sequence encoding the parent peptide may suitably be of genomic or cDNA origin, for instance obtained by preparing a genomic or cDNA library and screening for DNA sequences coding for all or part of the peptide by hybridisation using synthetic oligonucleotide probes in accordance with standard techniques (see, for example, Sambrook, J, Fritsch, EF and Maniatis, T, Molecular Cloning: A Laboratory Manual, Cold Spring Harbor Laboratory Press, New York, 1989). The DNA sequence encoding the peptide may also be prepared synthetically by established standard methods, e.g. the phosphoamidite method described by Beaucage and Caruthers, Tetrahedron Letters 22 (1981), 1859 - 1869, or the method described by Matthes et al., EMBO Journal 3 (1984), 801 - 805. The DNA sequence may also be prepared by polymerase chain reaction using specific primers, for instance as described in US 4,683,202 or Saiki et al., Science 239 (1988), 487 - 491.

The DNA sequence may be inserted into any vector which may conveniently be subjected to recombinant DNA procedures, and the choice of vector will often depend on the host cell into which it is to be introduced. Thus, the vector may be an autonomously replicating vector, *i.e.* a vector which exists as an extrachromosomal entity, the replication of which is independent of chromosomal replication, *e.g.* a plasmid. Alternatively, the vector may be one which, when introduced into a host cell, is integrated into the host cell genome and replicated together with the chromosome(s) into which it has been integrated.

The vector is preferably an expression vector in which the DNA sequence encoding the peptide is operably linked to additional segments required for transcription of the DNA, such as a promoter. The promoter may be any DNA sequence which shows transcriptional activity in the host cell of choice and may be derived from genes encoding proteins either homologous or heterologous to the host cell. Examples of suitable promoters for directing the transcription of the DNA encoding the peptide of the invention in a variety of host cells are well known in the art, cf. for instance Sambrook *et al., supra.*

The DNA sequence encoding the peptide may also, if necessary, be operably connected to a suitable terminator, polyadenylation signals, transcriptional enhancer sequences, and translational enhancer sequences. The recombinant vector of the invention may further comprise a DNA sequence enabling the vector to replicate in the host cell in question.

The vector may also comprise a selectable marker, *e.g.* a gene the product of which complements a defect in the host cell or one which confers resistance to a drug, *e.g.* ampicillin, kanamycin, tetracyclin, chloramphenicol, neomycin, hygromycin or methotrexate.

To direct a parent peptide of the present invention into the secretory pathway of the host cells, a secretory signal sequence (also known as a leader sequence, prepro sequence or pre sequence) may be provided in the recombinant vector. The secretory signal sequence is joined to the DNA sequence encoding the peptide in the correct reading frame. Secretory signal sequences are commonly positioned 5' to the DNA sequence encoding the peptide. The secretory signal sequence may be that normally associated with the peptide or may be from a gene encoding another secreted protein.

The procedures used to ligate the DNA sequences coding for the present peptide, the promoter and optionally the terminator and/or secretory signal sequence, respectively, and to insert them into suitable vectors containing the information necessary for replication, are well known to persons skilled in the art (cf., for instance, Sambrook *et al.., supra*).

The host cell into which the DNA sequence or the recombinant vector is introduced may be any cell which is capable of producing the present peptide and includes bacteria, yeast, fungi and higher eukaryotic cells. Examples of suitable host cells well known and used in the art are, without limitation, *E. coli, Saccharomyces cerevisiae,* or mammalian BHK or CHO cell lines.

The present invention is further illustrated by the following examples which, however, are not to be construed as limiting the scope of protection. The features disclosed in the foregoing description and in the following examples may, both separately and in any combination thereof, be material for realising the invention in diverse forms thereof.

## EXAMPLES

Below examples illustrate pharmaceutical compositions which are produced according to the invention.

Detemir designates the basal insulin having the structure : $N^{\varepsilon B29}$-tetradecanoyl des(B30) human insulin.

Liraglutide designates an insulinotropic peptide having the structure : $Arg^{34}$, $Lys^{26}(N^{\varepsilon}\text{-}(\gamma\text{-}Glu(N^{\alpha}\text{-}hexadecanoyl)))\text{-}GLP\text{-}1(7\text{-}37)$.

## General procedure for Thioflavin T (ThT) fibrillation assay:

### Principle

Low physical stability of a peptide may lead to amyloid fibril formation, which is observed as well-ordered, thread-like macromolecular structures in the sample eventually resulting in gel formation. This has traditionally been measured by visual inspection of the sample. However, that kind of measurement is very subjective and depending on the observer. Therefore, the application of a small molecule indicator probe is much more advantageous. Thioflavin T (ThT) is such a probe and has a distinct fluorescence signature when binding to fibrils [Naiki et al. (1989) Anal. Biochem. 177, 244-249; LeVine (1999) Methods. Enzymol. 309, 274-284].

The time course for fibril formation can be described by a sigmoidal curve with the following expression [Nielsen et al. (2001) Biochemistry 40, 6036-6046] :

$$F = f_i + m_i t + \frac{f_f + m_f t}{1 + e^{-[(t-t_0)/\tau]}} \quad \text{Eq.(1)}$$

Here, F is the ThT fluorescence at the time t. The constant $t_0$ is the time needed to reach 50% of maximum fluorescence. The two important parameters describing fibril formation are the lag-time calculated by $t_0 - 2\tau$ and the apparent rate constant $k_{app} = 1/\tau$.

Formation of a partially folded intermediate of the peptide is suggested as a general initiating mechanism for fibrillation. Few of those intermediates nucleate to form a template onto which further intermediates may assembly and the fibrillation proceeds. The lag-time corresponds to the interval in which the critical mass of nucleus is built up and the apparent rate constant is the rate with which the fibril itself is formed.

### Sample preparation

Samples were prepared freshly before each assay. Each sample composition is described in the legends. The pH of the sample was adjusted to the desired value using appropriate amounts of concentrated NaOH and $HClO_4$. Thioflavin T was added to the samples from a stock solution in $H_2O$ to a final concentration of $1\mu M$. Sample aliquots of 200 $\mu l$ were placed in a 96 well microtiter plate (Packard OptiPlate™-96, white polystyrene). Usually, eight replica of each sample (corresponding to one test condition) were placed in one column of wells. The plate was sealed with Scotch Pad (Qiagen).

### Incubation and fluorescence measurement

Incubation at given temperature, shaking and measurement of the ThT fluorescence emission were done in either a Fluoroskan Ascent FL or Varioskan fluorescence platereader (Thermo Labsystems). The temperature was adjusted to 37 °C. The orbital shaking was adjusted to 960 rpm with an amplitude of 1 mm in all the presented data. Fluorescence measurement was done using excitation through a 444 nm filter and measurement of emission through a 485 nm filter.

Each run was initiated by incubating the plate at the assay temperature for 10 min. The plate was measured every 20 minutes for typically 45 hours. Between each measurement, the plate was shaken and heated as described.

### Data handling

The measurement points were saved in Microsoft Excel format for further processing and curve drawing and fitting was performed using GraphPad Prism. The background emission from ThT in the absence of fibrils was negligible. The data points are typically a mean of eight samples and shown with standard deviation error bars. Only data obtained in the same experiment (i.e. samples on the same plate) are presented in the same graph ensuring a relative measure of fibrillation between the individual samples of one assay rather than comparison between different assays. The data set may be fitted to Eq. (1). However, since full sigmodial curves in this case are not usually achieved during the measurement time, the degree of fibrillation is expressed as ThT fluorescence at various time points calculated as the mean of the eight samples and shown with the standard deviation.

The following examples illustrate the invention.

## Example 1.

An example of a mixture of a basal insulin and liraglutide could be a preparation of detemir 1.2 mM and liraglutide 1.2 mM in a formulation comprising 0.5 mM Zn acetate, 8 mM sodium phosphate buffer, 20 mM sodium chloride, 60 mM phenol and 14 mg/ml propylene glycol at pH 7.7 with 200 ppm polysorbate 20.

## Example 2.

Another example of a mixture of a basal insulin and liralgutide could be a preparation of detemir 2.4 mM and liraglutide 3.0 mM in a formulation comprising 1.2 mM Zn acetate, 8 mM sodium phosphate buffer, 10 mM sodium chloride, 20 mM phenol, 20 mM *m*-cresol and 14 mg/ml propylene glycol at pH 7.7 with 300 ppm polysorbate 20.

## Example 3

Another example of a mixture of a basal insulin and liralgutide could be a preparation of detemir 1.2 mM and liraglutide 1.67 mM in a formulation comprising 0.8 mM Zn acetate, 8 mM glycyl glycine buffer, 10 mM sodium chloride, 20 mM phenol, 20 mM *m*-cresol and 14 mg/ml propylene glycol at pH 7.7 with 100 ppm poloxamer 188.

## Example 4

Another example of a mixture of a basal insulin and liralgutide could be a preparation of detemir 2.4 mM and liraglutide 1.2 mM in a formulation comprising 2.0 mM Zn acetate, 8 mM sodium phosphate buffer, 10 mM sodium chloride, 20 mM phenol, 20 mM *m*-cresol and 14 mg/ml propylene glycol at pH 7.7 with 300 ppm polysorbate 20.

## Example 5

Mixtures of detemir 2.4 mM and liraglutide 1.2 mM in a formulation comprising 1.2 mM Zn acetate, 5 mM glycyl glycine, 20 mM sodium chloride, 60 mM phenol and 14 mg/ml propylene glycol at pH 7.7 (figure 1, diamond) and the same formulation but with the addition of 300 ppm polysorbate 20 (tween 20). The ThT fluorescence was followed by the general procedure described above. This mixture is also shown with the addition of either 200 ppm Polysorbate-20 or 1000 ppm Poloxamer-188.

## Example 6

Mixtures of detemir 2.4 mM and liraglutide 1.2 mM in a formulation comprising 1.2 mM Zn acetate, 5 mM glycyl glycine, 20 mM sodium chloride, 60 mM phenol and 14 mg/ml propylene glycol at pH 7.7 (figure 2, diamond) and the same formulation but with the addition of 300 ppm polysorbate 20 (tween 20) at three different pH values, triangles (fig 2) at pH 7.4, squares (fig 2) at pH 7.7 and circles (fig 2) at pH 8.1. The ThT fluorescence was followed by the general procedure described above.

## Example 7

Mixtures of detemir 2.4 mM and liraglutide 2.4 mM in a formulation comprising 1.2 mM Zn acetate, 8 mM sodium phosphate buffer, 20 mM sodium chloride, 60 mM phenol and 14 mg/ml propylene glycol at pH 7.7 (figure 3, squares) and the same formulation but with the addition of 300 ppm polysorbate 20 shown with star symbols (fig 3). The ThT fluorescence was followed by the general procedure described above.

## Example 8

Mixtures of detemir 2.4 mM and liraglutide 2.4 mM in a formulation comprising 1.2 mM Zn acetate, 8 mM sodium phosphate buffer, 20 mM sodium chloride, 60 mM phenol and 14 mg/ml propylene glycol at pH 7.7 (figure 4, square) and the same formulation but with the addition of 2 levels of poloxamer 188, 100 ppm shown with crosses (fig 4) and 500 ppm with triangles (fig 4). The ThT fluorescence was followed by the general procedure described above.

## Example 9

Formulation A consists of: 0.6 mM $N^{\varepsilon B29}$-($N^{\alpha}$-(HOOC(CH$_2$)$_{14}$CO)-$\gamma$-Glu) desB30 human insulin, 0.3 mM $Zn^{2+}$ (corresponding to 3 $Zn^{2+}$ ions per insulin hexamer), 60 mM phenol, 14 mg/ml propylene glycol, 5 mM phosphate pH 7.7 and 1.2 mM liraglutide. This mixture starts to fibrillate with a short lag time of approximately one hour. However, the addition of either 200 ppm Polysorbate-20 or 1000 ppm Poloxamer-188 prolongs the lag time to more than 30 hours. (fig 5).

## Example 10

Formulation B consists of: 0.6 mM $N^{\varepsilon B29}$-($N^{\alpha}$-(HOOC(CH$_2$)$_{14}$CO)-$\gamma$-Glu) desB30 human insulin, 0.5 mM $Zn^{2+}$ (corresponding to 5 $Zn^{2+}$ ions per insulin hexamer), 60 mM phenol, 14 mg/ml propylene glycol, 5 mM phosphate pH 7.7 and 1.2 mM liraglutide. The same formulation shown with the addition of 500 ppm Polysorbate-20 and of 2000 ppm Poloxamer-188 (fig 6 ).

## Claims

1.  A shelf-stable pharmaceutical composition comprising an acylated GLP-1 analogue and a basal insulin, a pharmaceutically acceptable preservative, non-ionic surfactant, at a concentration of from 10 mg/L to 500 mg/L, and optionally a pharmaceutically acceptable tonicity modifier, where said composition has a pH that is in the range from 7.0 to 8.5, wherein said acylated GLP-1 analogue is $Arg^{34}$, $Lys^{26}(N^{\epsilon}$-($\gamma$-Glu($N^{\alpha}$-hexadecanoyl)))-GLP-1(7-37) and_said basal insulin is $N^{\epsilon B29}$-tetradecanoyl des(B30) human insulin or $N^{\epsilon B29}$-($N^{\alpha}$-(HOOC($CH_2$)$_{14}$CO)-$\gamma$-Glu) desB30 human insulin.

2. A pharmaceutical composition according to claim 1, wherein the surfactant is poloxamer 188.

3. A pharmaceutical composition according to claim 1, wherein the surfactant is selected from the group consisting of poloxamer 407, poloxamer 124, poloxamer 181, poloxamer 182, poloxamer 237, poloxamer 331 and poloxamer 338.

4. A pharmaceutical composition according to claim 1, wherein the surfactant is polysorbate 20.

5. A pharmaceutical composition according to any one of claims 1-4, further comprising an additional surfactant.

6. A pharmaceutical composition according to any one of the previous claims, wherein pH is in the range from 7.4 to 8.0.

7. A pharmaceutical composition according to any one of the previous claims, comprising a buffer which is a phosphate buffer.

8. A pharmaceutical composition according to any one of the previous claims wherein the concentration of said acylated GLP-1 analogue is in the range from 0.1 mg/mL to 25 mg/mL.

9. A pharmaceutical composition according to any one of the previous claims wherein the concentration of said acylated GLP-1 analogue is in the range from 1 mg/mL to 25 mg/mL.

10. A pharmaceutical composition according to any one of the previous claims wherein the concentration of said basal insulin is in the range from 1.6 mg/mL to 5.6 mg/mL.

**Patentansprüche**

1. Lagerstabile pharmazeutische Zusammensetzung, die ein acyliertes GLP-1-Analog und ein Basalinsulin, ein pharmazeutisch unbedenkliches Konservierungsmittel, nicht ionisches Tensid, in einer Konzentration von 10 mg/l bis 500 mg/l, sowie gegebenenfalls ein pharmazeutisch unbedenkliches Mittel zum Modifizieren der Tonizität umfasst, wobei die Zusammensetzung einen pH-Wert aufweist, der im Bereich von 7,0 bis 8,5 liegt, wobei es sich bei dem acylierten GLP-1-Analog um $Arg^{34}$, $Lys^{26}$ ($N^{\epsilon}$- ($\gamma$-Glu ($N^{\alpha}$-Hexadecanoyl)))-GLP-1(7-37) handelt und wobei es sich bei dem Basisinsulin um menschliches $N^{\epsilon B29}$-Tetradecanoyldes (B30) insulin oder um menschliches $N^{\epsilon B29}$- ($N^{\alpha}$- (HOOC ($CH_2$)$_{14}$CO)-$\gamma$-Glu) desB30-insulin handelt.

2. Pharmazeutische Zusammensetzung nach Anspruch 1, wobei es sich bei dem Tensid um Poloxamer 188 handelt.

3. Pharmazeutische Zusammensetzung nach Anspruch 1, wobei das Tensid aus der Gruppe bestehend aus Poloxamer 407, Poloxamer 124, Poloxamer 181, Poloxamer 182, Poloxamer 237, Poloxamer 331 und Poloxamer 338 ausgewählt ist.

4. Pharmazeutische Zusammensetzung nach Anspruch 1, wobei es sich bei dem Tensid um Polysorbat 20 handelt.

5. Pharmazeutische Zusammensetzung nach einem der Ansprüche 1-4, die weiterhin ein zusätzliches Tensid umfasst.

6. Pharmazeutische Zusammensetzung nach einem der vorhergehenden Ansprüche, wobei der pH-Wert im Bereich von 7,4 bis 8,0 liegt.

7. Pharmazeutische Zusammensetzung nach einem der vorhergehenden Ansprüche, die einen Puffer umfasst, bei dem es sich um einen Phosphatpuffer handelt.

8. Pharmazeutische Zusammensetzung nach einem der vorhergehenden Ansprüche, wobei die Konzentration des acylierten GLP-1-Analogs im Bereich von 0,1 mg/ml bis 25 mg/ml liegt.

9. Pharmazeutische Zusammensetzung nach einem der vorhergehenden Ansprüche, wobei die Konzentration des acylierten GLP-1-Analogs im Bereich von 1 mg/ml bis 25 mg/ml liegt.

10. Pharmazeutische Zusammensetzung nach einem der vorhergehenden Ansprüche, wobei die Konzentration des Basalinsulins im Bereich von 1,6 mg/ml bis 5,6 mg/ml liegt.

**Revendications**

1. Composition pharmaceutique stable à la conservation comprenant un analogue acylé de GLP-1 et une insuline basale, un conservateur pharmaceutiquement acceptable, un tensioactif non ionique, à une concentration de 10 mg/l à 500 mg/l, et facultativement un modificateur de tonicité pharmaceutiquement acceptable, où ladite composition a un pH qui est dans la plage de 7,0 à 8,5, où ledit analogue acylé de GLP-1 est Arg$^{34}$, Lys$^{26}$ (N$^{\epsilon}$- (y-Glu (N$^{\alpha}$-hexadécanoyl)))-GLP-1 (7-37) et ladite insuline basale est N$^{\epsilon B29}$-tétradécanoyldes (B30) -insuline humaine ou N$^{\epsilon B29}$- (N$^{\alpha}$-(HOOC(CH$_2$)$_{14}$CO)-$\gamma$-Glu)desB30-insuline humaine.

2. Composition pharmaceutique selon la revendication 1, dans laquelle le tensioactif est le poloxamère 188.

3. Composition pharmaceutique selon la revendication 1, dans laquelle le tensioactif est choisi dans le groupe constitué des poloxamère 407, poloxamère 124, poloxamère 181, poloxamère 182, poloxamère 237, poloxamère 331 et poloxamère 338.

4. Composition pharmaceutique selon la revendication 1, dans laquelle le tensioactif est le polysorbate 20.

5. Composition pharmaceutique selon l'une quelconque des revendications 1 à 4, comprenant en outre un tensioactif additionnel.

6. Composition pharmaceutique selon l'une quelconque des revendications précédentes, dans laquelle le pH est dans la plage de 7,4 à 8,0.

7. Composition pharmaceutique selon l'une quelconque des revendications précédentes, comprenant un tampon qui est un tampon phosphate.

8. Composition pharmaceutique selon l'une quelconque des revendications précédentes dans laquelle la concentration dudit analogue acylé de GLP-1 est dans la plage de 0,1 mg/ml à 25 mg/ml.

9. Composition pharmaceutique selon l'une quelconque des revendications précédentes dans laquelle la concentration dudit analogue acylé de GLP-1 est dans la plage de 1 mg/ml à 25 mg/ml.

10. Composition pharmaceutique selon l'une quelconque des revendications précédentes dans laquelle la concentration de ladite insuline basale est dans la plage de 1,6 mg/ml à 5,6 mg/ml.

Figure 1

| | 20 h | SD | 45 h | SD |
|---|---|---|---|---|
| 0 ppm polysorbate 20 | 226 | 25 | 230 | 15 |
| + 300 ppm polysorbate 20 | 3.0 | 0.1 | 2.5 | 0.1 |

Figure 2

|  | 20 h | SD | 45 h | SD |
|---|---|---|---|---|
| 0 ppm polysorbate 20 | 226 | 25 | 230 | 15 |
| + 300 ppm polysorbate 20 at pH 7.4 | 3.1 | 0.1 | 13 | 11 |
| + 300 ppm polysorbate 20 at pH 7.7 | 3.0 | 0.1 | 2.5 | 0.1 |
| + 300 ppm polysorbate 20 at pH 8.1 | 3.3 | 0.1 | 3.0 | 0.1 |

Figure 3

| | 10 h | SD | 40 h | SD |
|---|---|---|---|---|
| —□— 0 ppm polysorbate 20 | 284 | 48 | 267 | 25 |
| —✳— + 300 ppm polysorbate 20 | 2.1 | 0.1 | 252 | 10 |

Figure 4

| | 4 h | SD | 20 h | SD |
|---|---|---|---|---|
| 0 ppm poloxamer 188 | 283 | 71 | 274 | 32 |
| + 100 ppm poloxamer 188 | 27 | 50 | 247 | 32 |
| + 500 ppm poloxamer 188 | 3 | 2 | 273 | 37 |

Figure 5

| | 10 h | SD | 25 h | SD | 45 h | SD |
|---|---|---|---|---|---|---|
| Formulation A | 1405 | 169 | 1816 | 244 | 1775 | 333 |
| Formulation A + 200 ppm Polysorbate-20 | 22 | 0 | 25 | 3 | 321 | 270 |
| Formulation A + 1000ppm Poloxamer-188 | 39 | 1 | 41 | 2 | 83 | 48 |

Figure 6

| | 10 h | SD | 25 h | SD | 45 h | SD |
|---|---|---|---|---|---|---|
| Formulation B | 775 | 67 | 1189 | 132 | 1459 | 133 |
| Formulation B + 500 ppm Polysorbate-20 | 23 | 2 | 26 | 4 | 138 | 140 |
| Formulation B + 2000 ppm Poloxamer-188 | 26 | 0 | 26 | 0 | 28 | 2 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9808871 A
- WO 9943706 A
- US 4683202 A

### Non-patent literature cited in the description

- **SIEGEL et al.** *Regul. Pept.,* 1999, vol. 79, 93-102
- **MENTLEIN et al.** *Eur. J. Biochem.,* 1993, vol. 214, 829-35
- **REMINGTON.** The Science and Practice of Pharmacy. 1995
- **SAMBROOK, J ; FRITSCH, EF ; MANIATIS, T.** Molecular Cloning: A Laboratory Manual. Cold Spring Harbor Laboratory Press, 1989
- **BEAUCAGE ; CARUTHERS.** *Tetrahedron Letters,* 1981, vol. 22, 1859-1869
- **MATTHES et al.** *EMBO Journal,* 1984, vol. 3, 801-805
- **SAIKI et al.** *Science,* 1988, vol. 239, 487-491
- **NAIKI et al.** *Anal. Biochem.,* 1989, vol. 177, 244-249
- **LEVINE.** *Methods. Enzymol.,* 1999, vol. 309, 274-284
- **NIELSEN et al.** *Biochemistry,* 2001, vol. 40, 6036-6046